(19) Europäisches Patentamt
European Patent Office
Office européen des brevets

(11) Veröffentlichungsnummer: **0 033 088**
**B1**

# (12) EUROPÄISCHE PATENTSCHRIFT

(45) Veröffentlichungstag der Patentschrift: **25.05.83**

(51) Int. Cl.³: **A 61 K 31/41** // C07D249/08

(21) Anmeldenummer: **81100221.1**

(22) Anmeldetag: **14.01.81**

## (54) Antimykotische Mittel.

(30) Priorität: **24.01.80 DE 3002415**

(43) Veröffentlichungstag der Anmeldung: **05.08.81 Patentblatt 81/31**

(45) Bekanntmachung des Hinweises auf die Patenterteilung: **25.05.83 Patentblatt 83/21**

(84) Benannte Vertragsstaaten: **AT BE CH DE FR GB IT LI NL SE**

(56) Entgegenhaltungen:
**EP-A-0 007 505**
**EP-A-0 007 506**
**EP-A-0 007 707**

(73) Patentinhaber: **BAYER AG, Zentralbereich Patente, Marken und Lizenzen, D-5090 Leverkusen 1, Bayerwerk (DE)**

(72) Erfinder: **Kranz, Eckart, Dr., Am Acker 9, D-5600 Wuppertal 1 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**
Erfinder: **Haller, Ingo, Dr., Viktoriastrasse 99, D-5600 Wuppertal 1 (DE)**

EP 0 033 088 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Antimykotische Mittel

Die vorliegende Erfindung betrifft die Verwendung von neuen Triazolyl-alkandiol-Derivaten als Antimykotika.

Es ist bereits bekanntgeworden, daß 1-(β-Aryl)-ethyl-imdiazol-Derivate, wie insbesondere das 1-[2,4-Dichlor-β-(2,4-dichlorbenzyloxy)-phenethyl]-imidazolnitrat (MICONAZOL[R]), gute antimykotische Wirkung aufweisen (vgl. DE-AS 1 940 388). Jedoch ist deren Wirkung invivo, wie insbesondere gegen Candida, nicht immer voll befriedigend.

Es wurde gefunden, daß die Triazolyl-alkandiol-Derivate der allgemeinen Formel

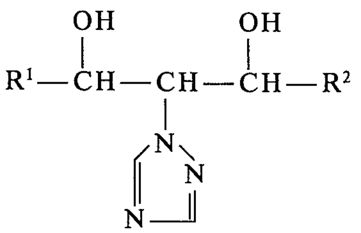

(II)

in welcher

$R^1$ für tert.-Butyl, sek.-Butyl, Isopropyl, Chlor-tert.-Butyl, Brom-tert.-butyl, Fluor-tert.-Butyl, 1,3-Dichlor-2-methyl-prop-2-yl, 2-Methylcarbamoyloxy-prop-2-yl, 2-Diethylcarbamoyloxy-prop-2-yl, Methylsulfonyloxy-tert.-butyl, Phenylsulfonyloxy-tert.-butyl, 4-Chlorphenyl-sulfonyloxy-tert.-butyl, Acetoxy-tert.-butyl, Ethylcarbonyloxy-tert.-butyl, Phenylcarbonyloxy-tert.-butyl, 4-Chlorphenyl-carbonyloxy-tert.-butyl, Diethylaminosulfonyloxy-tert.-butyl, Methoxy-tert.-butyl, Ethoxy-tert.-butyl, Isopropoxy-tert.-butyl, Phenoxy-tert.-butyl und 5-Chlorphenoxy-tert.-butyl steht; ferner für Phenyl, Chlorphenyl, Dichlorphenyl, Chlor-methyl-phenyl, Bromphenyl, Nitrophenyl, Biphenylyl, Nitro-biphenylyl, Chlorbiphenylyl, Phenoxyphenyl, Chlorphenoxyphenyl, Benzylphenyl, oder Chlorbenzylphenyl steht;

$R^2$ für Trichlormethyl, Dichlorfluormethyl, Trifluormethyl, Dichlormethyl, Chlormethyl, 1,1,2-Trichlorethyl, 1,1-Dibromethyl, 1,1-Dichlorethyl, 1,1,2-Trichlor-propyl, 2-Chlor-prop-2-yl, 1,2,2-Trichlorvinyl, 2,2-Dichlorvinyl, Methoxycarbonyl oder Ethoxycarbonyl steht,

und deren physiologisch verträglichen Säureadditionssalze und Metallsalzkomplexe gute antimykotische Eigenschaften aufweisen.

Die erfindungsgemäßen Verbindungen der Formel (I) enthalten asymmetrische Kohlenstoffatome; sie können deshalb in verschiedenen Diastereomeren vorliegen. Sie fallen im allgemeinen als Diastereomerengemische unterschiedlicher Zusammensetzung an. In allen Fällen liegen sie vorwiegend als Racemate vor.

Überraschenderweise zeigen die erfindungsgemäßen Triazolylalkandiol-Derivate neben einer guten antimykotischen in-vitro-Wirksamkeit eine bessere, therapeutisch nutzbare in-vivo-Wirksamkeit gegen Candida, als das aus dem Stand der Technik bekannte 1-[2,4-Dichlor-β-(2,4-dichlorbenzyloxy)-phenethyl]-imidazolnitrat, welches ein anerkanntes gutes Mittel gleicher Wirkungsrichtung ist. Die erfindungsgemäßen Wirkstoffe stellen somit eine wertvolle Bereicherung der Pharmazie dar.

Im einzelnen seien außer den bei den Herstellungsbeispielen angegebenen Verbindungen die folgenden Verbindungen der allgemeinen Formel (I) genannt:

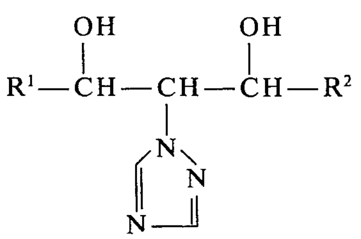

(I)

| $R^1$ | $R^2$ |
|---|---|
| $-C(CH_3)_3$ | $-CHCl_2$ |
| $-C(CH_3)_3$ | $-CO-OCH_3$ |

Fortsetzung

| $R^1$ | $R^2$ |
|---|---|
| $-C(CH_3)_3$ | $-CO-OC_2H_5$ |
| $-C(CH_3)_3$ | $-CO-OC_4H_9$ |
| $-C(CH_3)_3$ | $-CCl{=}CCl_2$ |
| $-C(CH_3)_3$ | $-CCl_2-CH_2Cl$ |
| $-C(CH_3)_2-CH_2Cl$ | $-CCl_2-CHCl-CH_3$ |
| $-C(CH_3)_2-CH_2Cl$ | $-CO-OCH_3$ |
| $-C(CH_3)_2-CH_2Cl$ | $-CCl_2-CH_2Cl$ |
| $-C(CH_3)_2-CH_2Cl$ | $-CO-OC_2H_5$ |
| $-C(CH_3)_2-CH_2Cl$ | $-CCl(CH_3)_2$ |
| $-C(CH_3)_2-CH_2Cl$ | $-CH{=}CCl_2$ |
| $-C(CH_3)_2-CH_2Cl$ | $-CCl{=}CCl_2$ |
| $-C(CH_3)_2-CH_2Br$ | $-CCl_3$ |
| $-C(CH_3)_2-CH_2Br$ | $-CO-OCH_3$ |
| $-C(CH_3)_2-CH_2Br$ | $-CH{=}CCl_2$ |
| $-C(CH_3)_2-CH_2Br$ | $-CCl{=}CCl_2$ |
| $-C(CH_3)_2-CH_2Br$ | $-CCl(CH_3)_2$ |
| $-C(CH_3)_2-CH_2Br$ | $-CHCl_2$ |
| $-C(CH_3)_2-CH_2Br$ | $-CCl_2-CHCl-CH_3$ |
| $-C(CH_3)_2-CH_2Br$ | $-CO-OC_2H_5$ |
| $-C(CH_3)_2-CH_2F$ | $-CO-OCH_3$ |
| $-C(CH_3)_2-CH_2F$ | $-CCl(CH_3)_2$ |
| $-C(CH_3)_2-CH_2F$ | $-CCl_2-CH_2Cl$ |
| $-C(CH_3)_2-CH_2F$ | $-CO-OC_2H_5$ |
| $-C(CH_3)_2-CH_2F$ | $-CH{=}CCl_2$ |
| $-C(CH_3)_2-CH_2F$ | $-CCl{=}CCl_2$ |
| 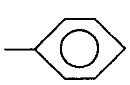 | $-CCl_3$ |
| 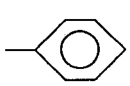 | $-CCl_2CHCl-CH_3$ |

Fortsetzung

| $R^1$ | $R^2$ |
|---|---|
| 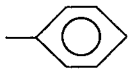 | $-CO-OC_2H_3$ |
| 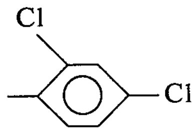 | $-CCl_2-CHCl-CH_3$ |
| 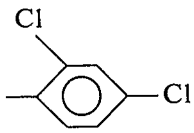 | $-CO-OC_2H_5$ |
| 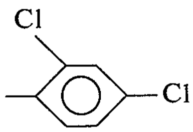 | $-CCl_2-CHCl-CH_3$ |
| 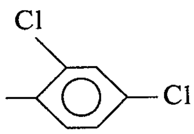 | $-CH{=}CCl_2$ |
| 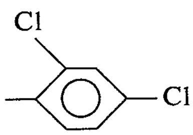 | $-CCl{=}CCl_2$ |
| 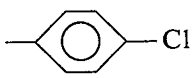 | $-CCl_2-CH_2Cl$ |
| 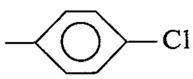 | $-CO-OC_2H_3$ |
| 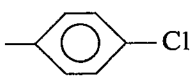 | $-CCl_2-CHCl-CH_3$ |
| 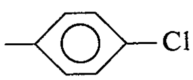 | $-CH{=}CCl_2$ |
| 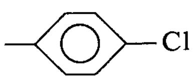 | $-CCl{=}CCl_2$ |
| 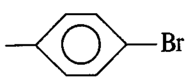 | $-CCl_3$ |
| 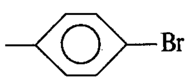 | $-CO-OC_2H_5$ |
| 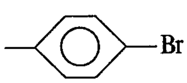 | $-CCl_2-CHCl-CH_3$ |
| 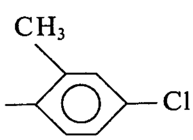 | $-CCl_3$ |

Fortsetzung

| $R^1$ | $R^2$ |
|---|---|
| 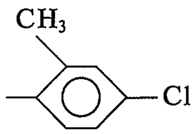 | $-CO-OC_2H_5$ |
| 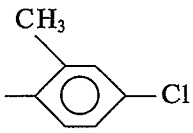 | $-CCl_2-CHCl-CH_3$ |
| 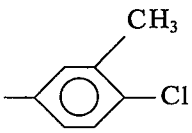 | $-CCl_3$ |
| 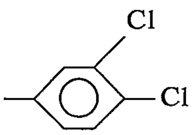 | $-CCl_5$ |
| 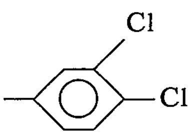 | $-CO-OC_2H_5$ |
| 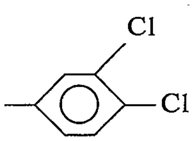 | $-CCl_2-CHCl-CH_3$ |
| 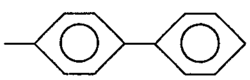 | $-CCl_3$ |
| 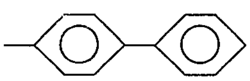 | $-CCl_2-CHCl-CH_3$ |
| 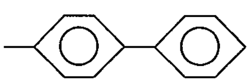 | $-CO-OC_2H_5$ |
| 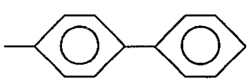 | $-CH=CCl_2$ |
| 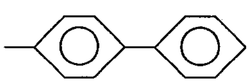 | $-CCl=CCl_2$ |
| 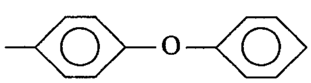 | $-CCl_3$ |
| 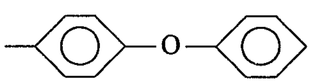 | $-CO-OC_2H_5$ |
| 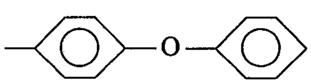 | $-CCl_2-CHCl-CH_3$ |
| 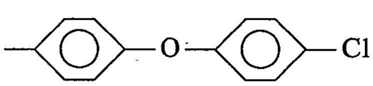 | $-CO-OC_2H_3$ |

Fortsetzung

| $R^1$ | $R^2$ |
|---|---|
| 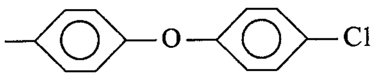 | $-CCl_2-CHCl-CH_3$ |
| 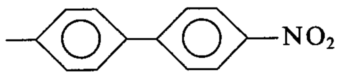 | $-CCl_3$ |
| 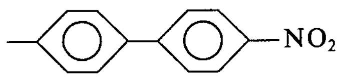 | $-CO-OC_2H_5$ |
| 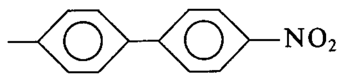 | $-CCl_2-CHCl-CH_3$ |
| $-C(CH_3)_2-O-CO-NHCH_3$ | $-CCl_3$ |
| $-C(CH_3)_2-O-CO-NHCH_3$ | $-CO-OC_2H_5$ |
| $-C(CH_3)_2-O-CO-NHCH_3$ | $-CCl_2-CHCl-CH_3$ |
| $-C(CH_3)_2-CH_2-O-SO_2CH_3$ | $-CCl_3$ |
| $-C(CH_3)_2-CH_2-O-SO_2CH_3$ | $-CO-OC_2H_5$ |
| $-C(CH_3)_2-CH_2-O-SO_2CH_3$ | $-CCl_2-CHCl-CH_3$ |
| $-C(CH_3)_2-CH_2-O-SO_2CH_3$ | $-CCl_2-CH_2Cl$ |
| 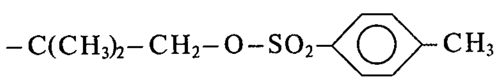 | $-CCl_3$ |
| 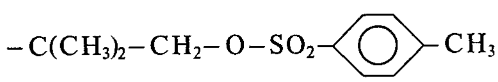 | $-CO-OC_2H_5$ |
| 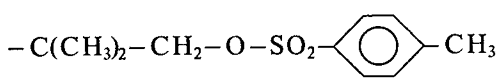 | $-CCl_2-CHCl-CH_3$ |
| $-C(CH_3)_2-CH_2-O-CO-CH_3$ | $-CCl_3$ |
| $-C(CH_3)_2-CH_2-O-CO-CH_3$ | $-CO-OC_2H_5$ |
| $-C(CH_3)_2-CH_2-O-CO-CH_3$ | $-CCl_2-CHCl-CH_3$ |
| $-C(CH_3)_2-OCH_3$ | $-CCl_3$ |
| $-C(CH_3)_2-OCH_3$ | $-CO-OC_2H_5$ |
| $-C(CH_3)_2-OCH_3$ | $-CCl_2-CHCl-CH_3$ |
| $-C(CH_3)_2-OC_2H_5$ | $-CCl_3$ |
| $-C(CH_3)_2-OC_2H_5$ | $-CO-OC_2H_5$ |
| $-C(CH_3)_2-OC_2H_5$ | $-CCl_2-CHCl-CH_3$ |
| 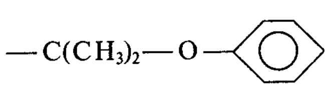 | $-CCl_3$ |

Fortsetzung

| $R^1$ | $R^2$ |
|---|---|
| 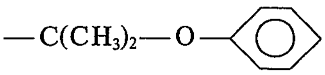 | $-CO-O-C_2H_5$ |
| 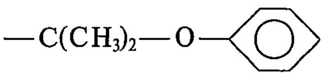 | $-CCl_2-CHCl-CH_3$ |

Die erfindungsgemäß zu verwendenden Wirkstoffe sind noch nicht bekannt. Sie können jedoch gemäß einem eigenen Vorschlag hergestellt werden, indem man $\alpha$-Triazolyl-$\beta$-hydroxy-ketone der Formel

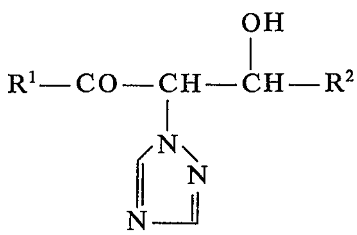 (II)

in welcher

$R^1$ und $R^2$ die oben angegebene Bedeutung haben,

nach bekannten Methoden reduziert, wie z. B. durch Umsetzung mit komplexen Hydriden, wie insbesondere Natriumborhydrid, gegebenenfalls in Gegenwart eines polaren Lösungsmittels, wie beispielsweise Alkohole, bei Temperaturen zwischen 0 und 30°C; oder durch Umsetzung mit Aluminiumsopropylat in Gegenwart eines polaren organischen Lösungsmittels, wie beispielsweise Isopropanol, bei Temperaturen zwischen 20 und 120°C. Die Aufarbeitung erfolgt in üblicher Weise; gegebenenfalls wird das Salz oder ein Metallkomplex hergestellt. In manchen Fällen erweist es sich als vorteilhaft, die Verbindungen der Formel (I) über ihre Salze in reiner Form zu erhalten.

Die $\alpha$-Triazolyl-$\beta$-hydroxy-ketone der Formel (II) sind noch nicht bekannt. Sie sind jedoch (mit) Gegenstand von eigenen älteren Anmeldungen [DE-OS 2 832 233 und DE-OS 2 944 447]. Sie werden erhalten, indem man $\alpha$-Triazolyl-ketone der Formel

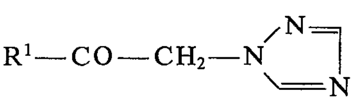 (III)

in welcher

$R^1$ die oben angegebene Bedeutung hat,

mit Aldehyden der Formel

$$R^2-CHO \qquad (IV)$$

in welcher

$R^2$ die oben angegebene Bedeutung hat,

in Gegenwart eines inerten organischen Lösungsmittels, wie beispielsweise Methylenchlorid und insbesondere Eisessig, und in Gegenwart eines Katalysators, wie beispielsweise Titantetrachlorid und insbesondere Natriumacetat, bei Temperaturen zwischen 20 und 100°C umsetzt (vgl. auch die Herstellungsbeispiele).

Die $\alpha$-Triazolyl-ketone der Formel (III) sind teilweise bekannt [vgl. DE-OS 2 063 857 und DE-OS 2 431 407]. Die noch nicht bekannten können nach den dort beschriebenen Verfahren erhalten werden, indem man z. B. entsprechende $\alpha$-Halogenketone mit 1,2,4-Triazol in Gegenwart eines inerten organischen Lösungsmittels, wie Aceton, und in Gegenwart eines Säurebindemittels, wie beispielsweise Natriumacetat, vorzugsweise in der Siedehitze umsetzt (vgl. auch die Herstellungsbeispiele).

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phsophorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäß verwendbaren Verbindungen der Formel (I), ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium coomune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise ausgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Springmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des

Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat, und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Meiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung, vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise parenteral, insbesondere intravenös, appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

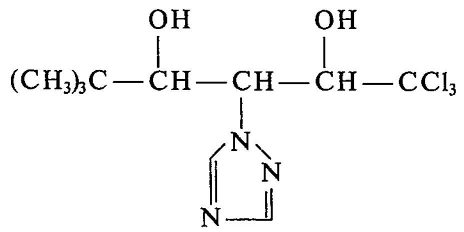

Zu einer Lösung von 31,5 g (0,1 Mol) 1,1,1-Trichlor-2-hydroxy-3-(1,2,4-triazol-1-yl)-5,5-dimethyl-hexan-4-on in 70 ml Wasser und 75 ml Dioxan werden bei Raumtemperatur unter Eiskühlung 4,4 g (0,12 Mol) Natriumborhydrid in 30 ml Wasser langsam zugetropft. Man läßt 10 Stunden bei Raumtemperatur nachrühren, versetzt mit 25 ml konzentrierter Salzsäure und läßt erneut ca. 2 Stunden bei Raumtemperatur nachrühren. Anschließend wird das Reaktionsgemisch in 500 ml gesättigte Natriumhydrogencarbonatlösung eingerührt. Man extrahiert dreimal mit je 100 ml Methylenchlorid, trocknet die vereinigten organischen Phasen über Natriumsulfat und engt durch Abdestillieren des Lösungsmittels im Vakuum ein. Der Rückstand wird in Methylenchlorid aufgenommen und mit Aktivkohle unter Rückfluß erhitzt. Das Gemisch wird filtriert und das Filtrat eingeengt. Man erhält 19,0 g (60% der Theorie) 1,1,1-Trichlor-3-(1,2,4-triazol-1-yl)-5,5-dimethyl-hexan-2,4-diol vom Schmelzpunkt 83 bis 113° C.

### Herstellung der Vorstufen

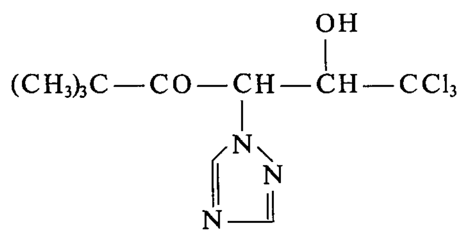

33,4 g (0,2 Mol) 3,3-Dimethyl-1-(1,2,4-triazol-1-yl)-butan-2-on werden in 150 ml Methylenchlorid gelöst und auf −5° C abgekühlt. Dazu tropft man langsam 19 g (0,1 Mol) Titantetrachlorid und anschließend 29,5 g (0,2 Mol) Chloral. Während der Zudosierung soll die Innentemperatur bei −5° C konstant bleiben. Danach wird langsam bis zum Rückfluß erwärmt und 3 Stunden verrührt. Die Reaktionslösung wird auf Eis gegossen und der entstehende weiße käsige Niederschlag abgesaugt. Nach Auskochen in 250 ml Methanol und anschließender Trocknung erhält man 56 g (90% der Theorie) 1,1,1-Trichlor-2-hydroxy-3-(1,2,4-triazol-1-yl)-5,5-dimethyl-hexan-4-on vom Schmelzpunkt 220 bis 222° C.

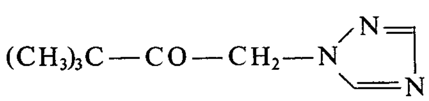

138 g (2 Mol) 1,2,4-Triazol werden bei Raumtemperatur portionsweise zu 276,4 g (2 Mol) gemahlenem Kaliumcarbonat und 269,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab.

Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72% der Theorie) 3,3-Dimethyl-1-(1,2,4-triazol-1-yl)-butan-2-on vom Schmelzpunkt 62 bis 64° C.

Beispiel 2

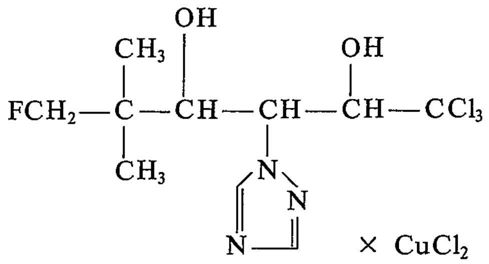

× $CuCl_2$

6,7 g (0,02 Mol) 1,1,1-Trichlor-3-(1,2,4-triazol-1-yl)-5,5-dimethyl-6-fluor-hexan-2,4-diol (Beispiel 7; Herstellung gemäß Beispiel 1) werden in 50 ml Ethanol suspendiert und mit 3,4 g (0,02 Mol) Kupfer-II-chlorid-dihydrat in 50 ml Ethanol 30 Minuten lang bei Raumtemperatur verrührt. Dabei erhält man eine klare, grüne Lösung, die durch Abdestillieren des Lösungsmittels im Vakuum eingeengt wird. Man erhält 8,7 g (93% der Theorie) 1,1,1-Trichlor-3-(1,2,4-triazol-1-yl)-5,5-dimethyl-6-fluorhexan-2,4-diol-kupfer-II-chlorid vom Schmelzpunkt 131 bis 140°C.

In entsprechender Weise und gemäß dem erfindungsgemäßen Verfahren werden die nachfolgenden Beispiele der allgemeinen Formel

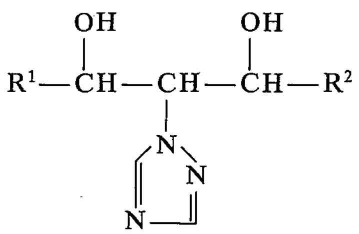

(I)

| Bsp. Nr. | $R^1$ | $R^2$ | Schmelzpunkt (°C) |
|---|---|---|---|
| 3 | $-C(CH_3)_3$ | $-CF_3$ | 134–44 |
| 4 | $-C(CH_3)_3$ | $-CCl(CH_3)_2$ | 120–22 |
| 5 | $-C(CH_3)_3$ | $-CCl_2-CH_2Cl$ | 57–63 |
| 6 | $-C(CH_3)_3$ | $-CH{=}CCl_2$ | Öl |
| 7 | $-C(CH_3)_2-CH_2F$ | $-CCl_3$ | Öl |
| 8 | $-C(CH_3)_2-CH_2Cl$ | $-CCl_3$ | 57–68 |
| 9 | $-C(CH_3)_2-CH_2F$ | $-CHCl_2$ | Öl |
| 10 | $-C(CH_3)_2-CH_2Cl$ | $-CHCl_2$ | 53–64 |
| 11 | $-C(CH_3)_2-CH_2F$ | $-CCl_2-CHCl-CH_3$ | Öl |
| 12 | 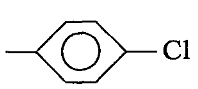 | $-CCl_3$ | 92–125 |
| 13 | 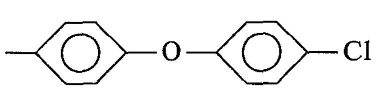 | $-CCl_3$ | Öl |
| 14 | 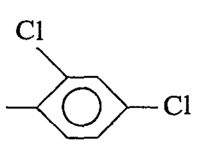 | $-CCl_3$ | 54–68 |

Fortsetzung

| Bsp. Nr. | $R^1$ | $R^2$ | Schmelzpunkt (°C) |
|---|---|---|---|
| 15 | 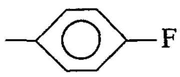 | $-CCl_3$ | Öl |
| 16 | $-C(CH_3)_3$ | $-COOCH_3$ | 82–84 |
| 17 | $-C(CH_3)_3$ | $-CCl_3$ | 162–69 (× $CuCl_2$) |
| 18 | $-C(CH_3)_3$ | $-CCl_3$ | 204–05 (× 2 $CuCl_2$) |
| 19 | $-C(CH_3)_3$ | $-CCl_2-CH_2Cl$ | 110–14 (× $CuCl_2$) |
| 20 | 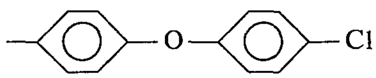 | $-CCl_3$ | 74–102 (× $CuCl_2$) |
| 21 | $-C(CH_3)_3$ | $-CCl_2-CHCl-CH_3$ | 54–57 |

## Verwendungsbeispiele

### Beispiel A

### Antimykotische in-vitro-Wirksamkeit

#### Versuchsbeschreibung

Die in-vitro-Prüfung im Reihenverdünnungstest mit Keiminokula von durchschnittlich 5 × 10 Keimen/ml Substrat durchgeführt. Als Nährmedium dienten

a) für Dermatophyten und Schimmelpilze:
Sabourand's milieu d'épresive;
b) für Hefen:
Isotonic Sensitest-Broth von Oxid.

Die Bebrütungstemperatur betrug 28° C; Bebrütungszeit war 24 Stunden bei Hefen und 96 Stunden bei Dermatophyten und Schimmelpilzen.

In diesen Tests zeigen die erfindungsgemäßen Mittel gute antimykotische in-vitro-Wirksamkeit.

### Beispiel B

### Antimikrobielle in-vivo-Wirksamkeit (oral) bei Mäuse-Candidose

#### Versuchsbeschreibung

Mäuse vom Typ SPF-$DF_1$ wurden intravenös mit 1 bis $2 \times 10^6$ logarythmisch wachsenden Candida-Zellen, die in physiologischer Kochsalzlösung suspendiert waren, infiziert. Eine Stunde vor und sieben Stunden nach der Infektion werden die Tiere mit jeweils 50 bis 100 mg/kg Körpergewicht der Präparate oral behandelt.

#### Ergebnis

Unbehandelte Tiere starben 3 bis 6 Tage post infektionem. Die Überlebensrate am 6. Tag post infektionem betrug bei unbehandelten Kontrolltieren etwa 5%.

Die erfindungsgemäßen Mittel der Beispiele 1, 4, 6, 11, 12, 14, 15 und 21 zeigen eine in-vivo-Wirksamkeit, die denjenigen der Verbindungen des Standes der Technik überlegen ist.

## Patentansprüche

1. Antimykotisches Mittel, gekennzeichnet durch einen Gehalt an mindestens einem Triazolyl-alkandiol-Derivat der allgemeinen Formel

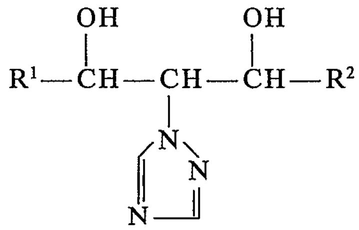

(II)

in welcher

$R^1$ für tert.-Butyl, sek.-Butyl, Isopropyl, Chlor-tert.-butyl, Brom-tert.-butyl, Fluor-tert.-butyl, 1,3-Dichlor-2-methyl-prop-2-yl, 2-Methylcarbamoyloxy-prop-2-yl, 2-Diethylcarbamoyloxy-prop-2-yl, Methylsulfonyloxy-tert.-butyl, Phenylsulfonyloxy-tert.-butyl, 4-Chlorphenylsulfonyloxy-tert.-butyl, Acetoxy-tert.-butyl, Ethylcarbonyloxy-tert.-butyl, Phenylcarbonyloxy-tert.-butyl, 4-Chlorphenylcarbonyloxy-tert.-butyl, Diethylaminosulfonyloxy-tert.-butyl, Methoxy-tert.-butyl, Ethoxy-tert.-butyl, Isopropoxy-tert.-butyl, Phenoxy-tert.-butyl und 4-Chlorphenoxy-tert.-butyl, Phenyl, Chlorphenyl, Dichlorphenyl, Chlor-methyl-phenyl, Bromphenyl, Nitrophenyl, Biphenylyl, Nitrobiphenylyl, Chlorbiphenylyl, Phenoxyphenyl, Chlorphenoxyphenyl, Benzylphenyl oder Chlorbenuylphenyl steht und

$R^2$ für Trichlormethyl, Dichlorfluormethyl, Trifluormethyl, Dichlormethyl, Chlormethyl, 1,1,2-Trichlorethyl, 1,1-Dibromethyl, 1,1-Dichlorethyl, 1,1,2-Trichlor-propyl, 2-Chlor-prop-2-yl, 1,2,2-Trichlorvinyl, 2,2-Dichlorvinyl, Methoxycarbonyl oder Ethoxycarbonyl steht,

und deren physiologische verträgliche Säureadditionssalze und Metallkomplexe.

2. Antimykotisches Mittel gemäß Anspruch 1, gekennzeichnet durch einen Gehalt an 1,1,1-Trichlor-3-(1,2,4-triazol-1-yl)-5,5-dimethyl-hexan-2,4-diol.

3. Antimykotisches Mittel gemäß Anspruch 1, gekennzeichnet durch einen Gehalt an 1-Chlor-1,1-dimethyl-3-(1,2,4-triazol-1-yl)-5,5-dimethyl-hexan-2,4-diol.

4. Antimykotisches Mittel gemäß Anspruch 1, gekennzeichnet durch einen Gehalt an 1,1,1-Trichlor-3-(1,2,4-triazol-1-yl)-4-(4'-chlorphenyl)-butan-2,4-diol.

5. Antimykotisches Mittel gemäß Anspruch 1, gekennzeichnet durch einen Gehalt an 1,1,1-Trichlor-3-(1,2,4-triazol-1-yl)-4-(2',4'-dichlorphenyl)-butan-2,4-diol.

6. Antimykotisches Mittel gemäß Anspruch 1, gekennzeichnet durch einen Gehalt an 1,1,1-Trichlor-3-(1,2,4-triazol-1-yl)-4-(4'-fluorphenyl)-butan-2,4-diol.

7. Verfahren zur Herstellung eines antimykotischen Mittels, dadurch gekennzeichnet, daß man Triazolylalkandiol Derivate gemäß der Formel in Anspruch 1 mit inerten, nicht toxischen, pharmazeutisch geeigneten Trägerstoffen vermischt.

## Claims

1. Antimycotic agent, characterised in that it contains at least one triazolyl-alkanediol derivative of the general formula

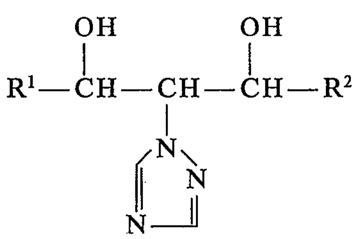

(II)

in which

$R^1$represents tert.-butyl, sec-butyl, isopropyl, chloro-tert.-butyl, bromo-tert.-butyl, fluoro-tert.-butyl, 1,3-dichloro-2-methyl-prop-2-yl, 2-methylcarbamoyloxy-prop-2-yl, 2-diethylcarbamoyloxy-prop-2-yl, methylsulphonyloxy-tert.-butyl, phenylsulphonyloxy-tert.-butyl, 4-chlorophenylsulphonyloxy-tert.-butyl, acetoxy-tert.-butyl, ethylcarbonyloxy-tert.-butyl, phenylcarbonyloxy-tert.-

butyl, 4-chlorophenylcarbonyloxy-tert.-butyl, diethylaminosulphonyloxy-tert.-butyl, methoxy-tert.-butyl, ethoxy-tert.-butyl, isopropoxy-tert.-butyl, phenoxy-tert.-butyl or 4-chlorophenoxy-tert.-butyl, or phenyl, chlorophenyl, dichlorophenyl, chloro-methyl-phenyl, bromophenyl, nitrophenyl, biphenylyl, nitrobiphenylyl, chlorobiphenylyl, phenoxyphenyl, chlorophenoxyphenyl, benzylphenyl or chlorobenzylphenyl, and

$R^2$ represents trichloromethyl, dichlorofluoromethyl, trifluoromethyl, dichloromethyl, chloromethyl, 1,1,2-trichloroethyl, 1,1-dibromoethyl, 1,1-dichloroethyl, 1,1,2-trichloro-propyl, 2-chloro-prop-2-yl, 1,2,2-trichlorovinyl; 2,2-dichlorovinyl, methoxycarbonyl or ethoxycarbonyl.

and physiologically acceptable acid addition salts and metal salt complexes thereof.

2. Antimycotic agent according to claim 1, characterised in that it contains 1,1,1-trichloro-3-(1,2,4-triazol-1-yl)-5,5-dimethyl-hexane-2,4-diol.

3. Antimycotic agent according to claim 1, characterised in that it contains 1-chloro-1,1-dimethyl-3-(1,2,4-triazol-1-yl)-5,5-dimethyl-hexane-2,4-diol.

4. Antimycotic agent according to claim 1, characterised in that it contains 1,1,1-trichloro-3-(1,2,4-triazol-1-yl)-4-(4′-chlorophenyl)-butane-2,4-diol.

5. Antimycotic agent according to claim 1, characterised in that it contains 1,1,1-trichloro-3-(1,2,4-triazol-1-yl)-4-(2′,4′-dichlorophenyl)-butane-2,4-diol.

6. Antimycotic agent according to claim 1, characterised in that it contains 1,1,1-trichloro-3-(1,2,4-triazol-1-yl)-4-(4′-fluorophenyl)-butane-2,4-diol.

7. Process for the preparation of an antimycotic agent, characterised in that triazolyl-alkanediol derivatives according to the formula in claim 1 are mixed with inert, non-toxic, pharmaceutically suitable excipients.

## Revendications

1. Agent antimycotique, caractérisé en ce qu'il contient au moins un dérivé de triazolyl-alcanediol de formule générale:

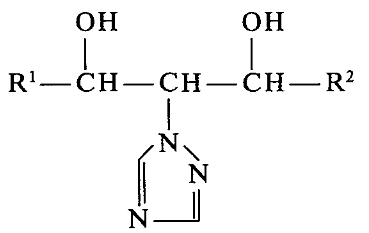

(I)

dans laquelle

$R^1$ représente un groupe tert-butyle, un groupe sec-butyle, un groupe isopropyle, un groupe chloro-tert-butyle, un groupe bromo-tert-butyle, un groupe fluoro-tert-butyle, un groupe 1,3-dichloro-2-méthyl-prop-2-yle, un groupe 2-méthylcarbamoyloxy-prop-2-yle, un groupe 2-diéthylcarbamoyloxyprop-2-yle, un groupe méthylsulfonyloxy-tert-butyle, un groupe phénylsulfonyloxy-tert-butyle, un groupe 4-chlorophénylsulfonyloxy-tert-butyle, un groupe acétoxy-tert-butyle, un groupe éthylcarbonyloxy-tert-butyle, un groupe phénylcarbonyloxy-tert-butyle, un groupe 4-chlorophénylcarbonyloxy-tert-butyle, un groupe diéthylaminosulfonyloxy-tert-butyle, un groupe méthoxy-tert-butyle, un groupe éthoxy-tert-butyle, un groupe isopropoxy-tert-butyle, un groupe phénoxy-tert-butyle ou un groupe 4-chlorophénoxy-tert-butyle, de même qu'un groupe phényle, un groupe chlorophényle, un groupe dichlorophényle, un groupe chlorométhylphényle, un groupe bromophényle, un groupe nitrophényle, un groupe diphénylyle, un groupe nitrodiphénylyle, un groupe chlorodiphénylyle, un groupe phénoxyphényle, un groupe chlorophénoxyphényle, un groupe benzylphényle ou un groupe chlorobenzylphényle et

$R^2$ représente un groupe trichlorométhyle, un groupe dichlorofluorométhyle, un groupe trifluorométhyle, un groupe dichlorométhyle, un groupe chlorométhyle, un groupe 1,1,2-trichloréthyle, un groupe 1,1-dibrométhyle, un groupe 1,1-dichloréthyle, un groupe 1,1,2-trichloropropyle, un groupe 2-chloroprop-2-yle, un groupe 1,2,2-trichlorovinyle, un groupe 2,2-dichlorovinyle, un groupe méthoxycarbonyle ou un groupe éthoxycarbonyle,

de même que les sels d'addition d'acide et les complexes métalliques physiologiquement compatibles de ce dérivé.

2. Agent antimycotique suivant la revendication 1, caractérisé en ce qu'il contient le 1,1,1-trichloro-3-(1,2,4-triazol-1-yl)-5,5-diméthyl-hexane-2,4-diol.

3. Agent antimycotique suivant la revendication 1, caractérisé en ce qu'il contient le 1-chloro-1,1-diméthyl-3-(1,2,4-triazol-1-yl)-5,5-diméthyl-hexane-2,4-diol.

4. Agent antimycotique suivant la revendication 1, caractérisé en ce qu'il contient le 1,1,1-trichloro-3-(1,2,4-triazol-1-yl)-4-(4'-chlorophényl)-butane-2,4-diol.
5. Agent antimycotique suivant la revendication 1, caractérisé en ce qu'il contient le 1,1,1-trichloro-3-(1,2,4-triazol-1-yl)-4-(2',4'-dichlorophényl)-butane-2,4-diol.
6. Agent antimycotique suivant la revendication 1, caractérisé en ce qu'il contient le 1,1,1-trichloro-3-(1,2,4-triazol-1-yl)-4-(4'-fluorophényl)-butane-2,4-diol.
7. Procédé de préparation d'un agent antimycotique, caractérisé en ce qu'on mélange des dérivés de triazolyl-alcane-diols répondant à la formule de la revendication 1 avec des substances supports inertes, non toxiques et pharmaceutiquement appropriées.